# EUROPEAN PATENT APPLICATION

(11) **EP 4 032 523 A1**
(43) Date of publication of application: **27.07.2022**
(21) Application number: 20865534.0
(22) Date of filing: 08.09.2020
(51) Int. Cl.: A61K 8/06, A61K 8/92, A61Q 1/08, A61Q 1/10

(54) **W/O TYPE EMULSION COSMETIC PREPARATION**

(30) Priority: 17.09.2019 JP 2019168788
(71) Applicant: Taiki Corp., Ltd., Osaka-shi, Osaka 534-0014 (JP)
(72) Inventor: IKEHARA, Yuriko, Osaka-shi, Osaka 534-0014 (JP); TAKAHASHI, Makoto, Osaka-shi, Osaka 534-0014 (JP); NAKAMURA, Koji, Osaka-shi, Osaka 534-0014 (JP)
(74) Representative: Grünecker Patent- und Rechtsanwälte PartG mbB
(86) International application number: PCT/JP2020/033909
(87) International publication number: WO 2021/054190

(57) **Abstract**

An object is to provide a W/0 type emulsion cosmetic composition that becomes a powder after being applied to the skin and, therefore, does not leave a sticky feel. A solution is to provide a W/0 type emulsion cosmetic composition including a volatile oil and a spherical powder, with a weight ratio between a total oil fraction and a total powder fraction (total powder fraction/total oil fraction) being 0.75 or greater and 1.40 or less. After being applied to the skin, the W/0 type emulsion cosmetic composition becomes a powder as a result of volatilization of a liquid.

## Description

### Technical Field

The present invention relates to a W/O type emulsion cosmetic composition.

### Background Art

W/O type (water-in-oil type) emulsion cosmetic compositions have characteristics such as high resistance to sweat or water and excellent long wearability. However, since the external phase is made of oil, W/O type emulsion cosmetic compositions tend to leave a "sticky feel" compared with O/W type (oil-in-water type) emulsion cosmetic compositions. To inhibit the sticky feel due to a W/O type emulsion cosmetic composition, an attempt that has been made is to include a volatile oil and a powder therein. For example, PTL 1 proposes a W/O type make-up cosmetic composition that does not impart an oily feel, which includes a particulate powder having a primary particle diameter within a range of 5 to 350 nm, with the powder being strongly dispersed in a solvent that includes at least one of a volatile silicone and a light liquid isoparaffin.

Furthermore, in recent years, W/O type liquid foundations, which are easy to apply to the skin and have excellent coverage, have been increasingly popular; however, a dry feel after application is desired.

### Citation List

### Patent Literature

PTL 1: Japanese Unexamined Patent Application Publication No. 8-157327

### Summary of Invention

### Technical Problem

An object of the present invention is to provide a W/O type emulsion cosmetic composition that becomes a powder after being applied to the skin and, therefore, does not leave a sticky feel.

### Solution to Problem

Means for achieving the object of the present invention are as follows.
1. A W/O type emulsion cosmetic composition including a volatile oil and a spherical powder, characterized in that
   a weight ratio between a total oil fraction and a total powder fraction (total powder fraction/total oil fraction) is 0.75 or greater and 1.40 or less, and
   after being applied to a skin, the W/O type emulsion cosmetic composition becomes a powder as a result of volatilization of a liquid.
2. The W/0 type emulsion cosmetic composition according to 1, characterized in that the volatile oil includes at least one of cyclic silicone oils, linear or branched silicone oils, and paraffinic hydrocarbon oils.
3. The W/0 type emulsion cosmetic composition according to 1 or 2, characterized in that the spherical powder has a volume average particle diameter of 5 µm or greater and 20 µm or less.
4. The W/0 type emulsion cosmetic composition according to any one of 1 to 3, characterized in that a percentage of the volatile oil in the total oil fraction (volatile oil/total oil fraction) is 30 wt.% or greater.
5. The W/0 type emulsion cosmetic composition according to any one of 1 to 4, characterized in that a percentage of the spherical powder in the total powder fraction (spherical powder/total powder fraction) is 45 wt.% or greater. Advantageous Effects of Invention

The W/0 type emulsion cosmetic composition of the present invention is in a liquid form during application to the skin and, therefore, can be spread uniformly. The W/0 type emulsion cosmetic composition of the present invention becomes a powder after being applied to the skin and, therefore, does not leave a sticky feel and provides an excellent dry feel. The W/0 type emulsion cosmetic composition of the present invention adheres to the skin and, therefore, has excellent long wearability.

### Description of Embodiments

A W/O type emulsion cosmetic composition of the present invention includes a volatile oil and a spherical powder and is characterized in that a weight ratio between a total oil fraction and a total powder fraction (total powder fraction/total oil fraction) is 0.75 or greater and 1.40 or less, and after being applied to the skin, the W/O type emulsion cosmetic composition becomes a powder as a result of volatilization of a liquid. Because of the weight ratio between the total oil fraction and the total powder fraction of 0.75 or greater and 1.40 or less, the resulting W/O type emulsion cosmetic composition can become a powder after being applied to the skin and, therefore, provide a dry feel. The weight ratio between the total oil fraction and the total powder fraction is preferably 0.80 or greater and 1.30 or less and more preferably 0.90 or greater and 1.10 or less.

In the present invention, the "volatile oil" refers to an oil having a boiling point at 1 atmosphere of 270°C or less. Examples of the volatile oil to be used in the present invention include, but are not limited to, cyclic silicone oils, such as octamethylcyclotetrasiloxane, decamethylcyclopentasiloxane, and dodecamethylcyclohexasiloxane; linear or branched silicone oils, such as dimethylpolysiloxane (dimethicone) having a low degree of polymerization, methyl trimethicone, and dimethylpolysiloxane; and hydrocarbon oils, examples of which include paraffinic hydrocarbon oils, such as isododecane, isodecane, isohexadecane, n-decane, n-undecane, and n-dodecane, isoparaffinic hydrocarbon oils, such as cyclodecane, light liquid isoparaffins, and hydrogenated polyisobutene, and cyclic paraffinic hydrocarbon oils, such as cyclododecane. One or more of these may be used. Among these, cyclic silicone oils, linear or branched silicone oils, and paraffinic hydrocarbon oils are preferable because these oils have good compatibility with other ingredients and provide a light feel when applied to the skin.

The W/O type emulsion cosmetic composition of the present invention may include an oil other than the volatile oil. The oil other than the volatile oil is not particularly limited provided that a uniform oil phase can be formed in instances in which the oil is mixed with the volatile oil. The oil may be a solid oil, a semi-solid oil, and/or a liquid oil. The oil other than the volatile oil affects the spreadability and feel associated with the application to the skin. One such oil or a mixture of two or more such oils may be used. Note that the solid oil is an oil in a solid form at room temperature (25°C), and the liquid oil is an oil in a liquid form at room temperature (25°C). The semi-solid oil is an oil having a melting point at a temperature above room temperature (25°C) and, therefore, is not completely solidified at room temperature; the semi-solid oil is an oil that is distinguished from the liquid oil and the solid oil.

Examples of the solid oil include cholesteryl stearate, diglyceryl stearate, glyceryl tristearate, chimyl stearate, beeswaxes, linear higher alcohols, batyl alcohol, and hydroxystearic acid.

Examples of the semi-solid oil include petrolatums, hydrogenated castor oil stearate, hydrogenated jojoba oils, and caprylic/capric triglycerides.

Examples of the liquid oil include squalane, silicone oils, liquid paraffins, isopropyl myristate, jojoba oils, olive oils, macadamia nut oils, synthetic isoparaffins, diisopropyl adipate, glyceryl tri-2-ethylhexanoate, octydodecyl myristate, and isostearyl alcohols.

In instances where the W/O type emulsion cosmetic composition of the present invention includes an oil other than the volatile oil, it is preferable that a percentage of the volatile oil in the total oil fraction (volatile oil/total oil fraction) be greater than or equal to 30 wt.%. When the percentage of the volatile oil is greater than or equal to 30 wt.%, the resulting W/O type emulsion cosmetic composition is easy to apply and provides an excellent dry feel and finish. The upper limit of the percentage of the volatile oil is not particularly limited. In terms of preventing non-uniformity associated with the application to the skin, it is preferable that the upper limit be less than or equal to 90 wt.%.

In the present invention, the "spherical powder" may or may not have an exactly spherical shape; the spherical powder may have a shape such as elliptical or substantially spherical or a shape having microscopic holes or irregularities in a surface. The spherical powder may be a spherical powder having pores in a surface or an interior thereof. In instances where the spherical powder is elliptical or substantially spherical, it is preferable that a ratio of a minor axis length to a major axis length be less than or equal to 1:3. More preferably, the ratio is less than or equal to 1:2.

The spherical powder used in the present invention may include organic particles or inorganic particles, which may be, for example, particles of silica, nylon, polyethylene, a vinyl dimethicone/methicone silsesquioxane crosspolymer, polymethylsilsesquioxane, polymethylmethacrylate, a methyl methacrylate crosspolymer, or the like. One or more of these may be used.

It is preferable that the spherical powder have a volume average particle diameter of 5 µm or greater and 20 µm or less. When the volume average particle diameter of the spherical powder is within the range, an excellent dry feel after application to the skin is achieved. As used herein, the volume average particle diameter is a value measured by a laser diffraction/scattering particle size distribution analyzer.

The W/O type emulsion cosmetic composition of the present invention may include a powder other than the spherical powder. Examples of the powder include plate-like powders, inorganic color pigments, and organic color pigments. The plate-like powders may be powders of talc, mica, sericite, kaolin, isinglass, an amino-acid system, or the like; one or more such powders may be used. The inorganic color pigments and the organic color pigments may be those used in the field of cosmetics; one or more such pigments may be used.

In instances where the W/O type emulsion cosmetic composition of the present invention includes a powder other than the spherical powder, it is preferable that a percentage of the spherical powder in the total powder fraction (spherical powder/total powder fraction) be greater than or equal to 45 wt.%. When the percentage of the spherical powder is greater than or equal to 45 wt.%, the resulting W/O type emulsion cosmetic composition provides an excellent dry feel. The upper limit of the percentage of the spherical powder is not particularly limited.

The present invention relates to W/O type emulsion cosmetic compositions and may include any of a variety of additives that are used in cosmetics, as long as a W/O type emulsion can be formed. Examples of the additives include medicinal components, surfactants, thickening agents, humectants, antioxidants, UV absorbing agents, antiinflammatory agents, vitamins, bactericides, coloring agents, deodorants, and perfumes.

Uses of the W/O type emulsion cosmetic compositions of the present invention are not particularly limited. For example, the uses may be those in foundations, makeup bases, blushers, or eye shadows.

### EXAMPLES

### Examples 1 to 7 and Comparative Examples 1 to 3

The ingredients listed below were combined and subjected to a commonly used emulsification and dispersion process. Accordingly, W/O type emulsion cosmetic compositions were obtained. The compounding amounts (weight ratio) of the ingredients are shown in Table 1 below.

Water: ion-exchanged water
Humectant
   Glycerin: concentrated glycerin for cosmetics
   PEG-150: PEG-6000
   Propanediol: propanediol
Chelating agent
   EDTA-2Na: Chelest 2BS
Preservative
   Phenoxyethanol: phenoxyethanol

### Thickening agent

Magnesium aluminum silicate: Sumecton SA
Distearyldimonium chloride, isopropanol: Cation DSV Surfactant
PEG-10 dimethicone: silicone KF-6017
Sorbitan sesquiisostearate: Cosmol 182V

### Oil

Cyclopentasiloxane: KF-995 (volatile oil)
Diphenylsiloxy phenyl trimethicone: silicone KF-56A
Dimethicone 1: silicone KF-96A-6cs
Triethylhexanoin: NIKKOL Trifat S-308

### Extender pigment

Talc, methicone: SI-2 TALC JA-46R
Mica, methicone: SI-2 MICA Y-2300

### Color pigment

Titanium oxide, aluminum hydroxide, methicone: SI-2 TiO₂ CR-50
Iron oxide, methicone: SI-2 YELLOW LL-100P

### Organic pigment

Red 226, barium sulfate, talc: Red No. 226 TA-20 Feel-improving powder
Methyl methacrylate crosspolymer: GMX-0810 (spherical powder 8 µm)

The following evaluation was performed on the obtained W/O type emulsion cosmetic compositions. The results are shown in Table 1.

### -Sensory evaluation

Ten trained panelists performed a sensory evaluation for "ease of application", a "dry feel", a "finish", and "long wearability", according to the following criteria.
5: Good
4: Fair
3: Ordinary
2: Slightly poor
1: Poor

An average of the ratings given by the ten panelists according to the above criteria was assessed according to the assessment criteria below. In instances where an assessment of "Δ" or "×" was given for two or more categories, the example was designated as a Comparative Example.
⊙: 4 or more
O: 3 or more and less than 4
Δ: 2 or more and less than 3
×: less than 2

**[Table 1]**

| | Label name | Example 1 | Example 2 | Example 3 | Example 4 | Example 5 | Example 6 | Example 7 | Comparative example 1 | Comparative example 2 | Comparative example 3 |
|---|---|---|---|---|---|---|---|---|---|---|---|
| Water | water | 40.58 | 30.95 | 35.95 | 32.95 | 34.45 | 40.95 | 28.23 | 25.95 | 42.65 | 24.37 |
| Humectant | glycerin | 2.00 | ← | ← | ← | ← | ← | ← | ← | ← | ← |
| | PEG-150 | 0.50 | ← | ← | ← | ← | ← | ← | ← | ← | ← |
| | propanediol | 7.00 | ← | ← | ← | ← | ← | ← | ← | ← | ← |
| Chelating agent | EDTA-2Na | 0.05 | ← | ← | ← | ← | ← | ← | ← | ← | ← |
| Preservative | phenoxyethanol | 0.50 | ← | ← | ← | ← | ← | ← | ← | ← | ← |
| Thickening agent | magnesium aluminum silicate | 0.68 | ← | ← | ← | ← | ← | ← | ← | ← | ← |
| | distearyldimonium chloride, isopropanol | 0.23 | ← | ← | ← | ← | ← | ← | ← | ← | ← |
| Surfactant | PEG-10 dimethicone | 2.00 | ← | ← | ← | ← | ← | ← | ← | ← | ← |
| | sorbitan sesquiisostearate | 1.00 | ← | ← | ← | ← | ← | ← | ← | ← | ← |
| Oil | cyclopentasiloxane (volatile oil) | 13.77 | 18.40 | 18.40 | 21.40 | 19.90 | 18.40 | 26.13 | 18.40 | 11.70 | 29.98 |
| | diphenylsiloxy phenyl trimethicone | 3.00 | ← | ← | ← | ← | ← | ← | ← | ← | ← |
| | dimethicone 1 | 1.00 | ← | ← | ← | ← | ← | ← | ← | ← | ← |
| | triethylhexanoin | 2.00 | ← | ← | ← | ← | ← | ← | ← | ← | ← |
| Extender pigment (powder) | talc, methicone | 3.00 | ← | ← | ← | ← | ← | ← | ← | ← | ← |
| | mica, methicone | 2.00 | ← | ← | ← | ← | ← | ← | ← | ← | ← |
| Color pigment (powder) | titanium oxide, aluminum hydroxide, methicone | 0.10 | ← | ← | ← | ← | ← | ← | ← | ← | ← |
| | iron oxide, methicone | 0.10 | ← | ← | ← | ← | ← | ← | ← | ← | ← |
| Organic pigment (powder) | Red 226, barium sulfate, talc | 0.50 | ← | ← | ← | ← | ← | ← | ← | ← | ← |
| Feel-improving powder | methyl methacrylate crosspolymer (spherical powder) | 20.00 | 25.00 | 20.00 | 20.00 | 20.00 | 15.00 | 20.00 | 30.00 | 20.00 | 20.00 |
| Total | | 100.0 | 100.0 | 100.0 | 100.0 | 100.0 | 100.0 | 100.0 | 100.0 | 100.0 | 100.0 |
| Weight ratio of total powder fraction to total oil fraction | | 1.30 | 1.26 | 1.05 | 0.94 | 0.99 | 0.85 | 0.80 | 1.46 | 1.45 | 0.71 |
| Ease of application | | ○ | Δ | ○ | ○ | ⊙ | Δ | ⊙ | × | × | Δ |
| Dry feel | | ○ | ○ | ⊙ | ⊙ | ⊙ | ⊙ | ⊙ | Δ | Δ | Δ |
| Finish | | ⊙ | ○ | ⊙ | ⊙ | ⊙ | ○ | ⊙ | Δ | Δ | Δ |
| Long wearability | | ⊙ | ⊙ | ⊙ | ⊙ | ⊙ | ⊙ | ⊙ | Δ | ○ | ○ |

The W/O type emulsion cosmetic compositions obtained in Examples 1 to 7, which are encompassed by the present invention, were easy to apply and had an excellent dry feel and finish and excellent long wearability.

The W/O type emulsion cosmetic compositions obtained in Comparative Examples 1 and 2, in which the weight ratio of the total powder fraction to the total oil fraction was greater than that of the present invention (the quantity of powders was larger), were difficult to apply and were somewhat inferior in terms of a dry feel.

The W/O type emulsion cosmetic composition obtained in Comparative Example 3, in which the weight ratio of the total powder fraction to the total oil fraction was smaller than that of the present invention (the quantity of oils was larger), was somewhat inferior in terms of ease of application, a dry feel, and a finish, and in addition, took a long time to vaporize after application.

### Examples 8 and 9 and Comparative Examples 4 to 6

W/O type emulsion cosmetic compositions were prepared in which the percentage of the volatile oil in the total oil fraction (volatile oil/total oil fraction) and the percentage of the spherical powder in the total powder fraction (spherical powder/total powder fraction) were adjusted with respect to the W/0 type emulsion cosmetic composition obtained in Example 5, which had an excellent rating in each of the evaluations as shown in Table 1 and was used as a reference. The evaluations were then performed. The compounding amounts and evaluation results are shown in Table 2.

**[Table 2]**

| | Label name | Example 5 | Example 8 | Example 9 | Comparative example 4 | Comparative example 5 | Comparative example 6 |
|---|---|---|---|---|---|---|---|
| Water | water | 34.45 | ← | ← | ← | ← | ← |
| Humectant | glycerin | 2.00 | ← | ← | ← | ← | ← |
| | PEG-150 | 0.50 | ← | ← | ← | ← | ← |
| | propanediol | 7.00 | ← | ← | ← | ← | ← |
| Chelating agent | EDTA-2Na | 0.05 | ← | ← | ← | ← | ← |
| Preservative | phenoxyethanol | 0.50 | ← | ← | ← | ← | ← |
| Thickening agent | magnesium aluminum silicate | 0.68 | ← | ← | ← | ← | ← |
| | distearyldimonium chloride, isopropanol | 0.23 | ← | ← | ← | ← | ← |
| Surfactant | PEG-10 dimethicone | 2.00 | ← | ← | ← | ← | ← |
| | sorbitan sesquiisostearate | 1.00 | ← | ← | ← | ← | ← |
| Oil | cyclopentasiloxane (volatile oil) | 19.90 | 9.90 | 19.90 | 6.90 | 1.90 | 19.90 |
| | diphenylsiloxy phenyl trimethicone | 3.00 | ← | ← | ← | ← | ← |
| | dimethicone 1 | 1.00 | ← | ← | ← | ← | ← |
| | triethylhexanoin | 2.00 | 12.00 | 2.00 | 15.00 | 20.00 | 2.00 |
| Extender pigment (powder) | talc, methicone | 3.00 | 3.00 | 10.00 | 3.00 | 3.00 | 12.00 |
| | mica, methicone | 2.00 | ← | ← | ← | ← | ← |
| Color pigment (powder) | titanium oxide, aluminum hydroxide, methicone | 0.10 | ← | ← | ← | ← | ← |
| | iron oxide, methicone | 0.10 | ← | ← | ← | ← | ← |
| Organic pigment (powder) | Red 226, barium sulfate, talc | 0.50 | ← | ← | ← | ← | ← |
| Feel-improving powder | methyl methacrylate crosspolymer (spherical powder) | 20.00 | 20.00 | 13.00 | 20.00 | 20.00 | 11.00 |
| Total | | 100.0 | 100.0 | 100.0 | 100.0 | 100.0 | 100.0 |
| Weight ratio of total powder fraction to total oil fraction | | 0.99 | 0.99 | 0.99 | 0.99 | 0.99 | 0.99 |
| Percentage of volatile oil in total oil fraction (wt.%) | | 76.8 | 38.2 | 76.8 | 26.6 | 7.3 | 76.8 |
| Percentage of spherical powder in total powder fraction (wt.%) | | 77.8 | 77.8 | 50.6 | 77.8 | 77.8 | 42.8 |
| Ease of application | | ⊙ | ○ | ○ | Δ | × | Δ |
| Dry feel | | ⊙ | ⊙ | ⊙ | Δ | × | × |
| Finish | | ⊙ | ⊙ | ⊙ | Δ | Δ | Δ |
| Long wearability | | ⊙ | ⊙ | ⊙ | ○ | Δ | ○ |

### Examples 10 to 14 and Comparative Examples 7 and 8

W/0 type emulsion cosmetic compositions were prepared in which one or more of the feel-improving powders listed below were added, and an evaluation of a dry feel was performed. The compounding amounts and evaluation results are shown in Table 3.

### Feel-improving powder

Methyl methacrylate crosspolymer: GMX-0810 (spherical powder 8 µm)
Nylon-6: coupler-6 (spherical powder 20 µm)
Methyl methacrylate crosspolymer: Makibiads 80 (spherical powder, porous, 7 µm)
Silica: Sunsphere L51 (spherical powder, 5 µm)
Lauroyl lysine: Amihope LL (plate-like powder)

**[Table 3]**

| Classification | Label name | Example 10 | Example 11 | Example 12 | Example 13 | Example 14 | Comparative example 7 | Comparative example 8 |
|---|---|---|---|---|---|---|---|---|
| Water | water | 15.95 | 15.95 | 15.95 | 15.95 | 35.95 | 15.95 | 15.95 |
| Humectant | glycerin | 2.00 | ← | ← | ← | ← | ← | ← |
| | PEG-150 | 0.50 | ← | ← | ← | ← | ← | ← |
| | propanediol | 7.00 | ← | ← | ← | ← | ← | ← |
| Chelating agent | EDTA-2Na | 0.05 | ← | ← | ← | ← | ← | ← |
| Preservative | phenoxyethanol | 0.50 | ← | ← | ← | ← | ← | ← |
| Thickening agent | magnesium aluminum silicate | 0.90 | ← | ← | ← | 0.68 | 0.90 | ← |
| | distearyldimonium chloride, isopropanol | 0.60 | ← | ← | ← | 0.23 | 0.60 | ← |
| Surfactant | PEG-10 dimethicone | 2.00 | ← | ← | ← | ← | ← | ← |
| | sorbitan sesquiisostearate | 1.00 | ← | ← | ← | ← | ← | ← |
| Oil | cyclopentasiloxane (volatile oil) | 28.80 | 28.80 | 28.80 | 28.80 | 18.40 | 28.80 | 28.80 |
| | diphenylsiloxy phenyl trimethicone | 3.00 | ← | ← | ← | ← | ← | ← |
| | dimethicone 1 | | | | | 1.00 | | |
| | triethylhexanoin | 2.00 | ← | ← | ← | ← | ← | ← |
| Extender pigment (powder) | talc, methicone | 3.00 | ← | ← | ← | ← | ← | 18.00 |
| | mica, methicone | 2.00 | ← | ← | ← | ← | ← | ← |
| Color pigment (powder) | titanium oxide, aluminum hydroxide, methicone | 0.10 | ← | ← | ← | ← | ← | ← |
| | iron oxide, methicone | 0.10 | ← | ← | ← | ← | ← | ← |
| Organic pigment (powder) | Red 226, barium sulfate, talc | 0.50 | ← | ← | ← | ← | ← | ← |
| Feel-improving powder | methyl methacrylate crosspolymer (spherical powder) | 30.00 | 15.00 | 15.00 | 15.00 | | 15.00 | 15.00 |
| | nylon-6 (spherical powder) | | 15.00 | | | | | |
| | methyl methacrylate crosspolymer (spherical powder) | | | 15.00 | | | | |
| | silica (spherical powder) | | | | 15.00 | 20.00 | | |
| | lauroyl lysine | | | | | | 15.00 | |
| Total | | 100.0 | 100.0 | 100.0 | 100.0 | 100.0 | 100.0 | 100.0 |
| Weight ratio of total powder fraction to total oil fraction | | 1.06 | 1.06 | 1.06 | 1.06 | 1.05 | 1.06 | 1.06 |
| Percentage of volatile oil in total oil fraction (wt.%) | | 85.2% | 85.2% | 85.2% | 85.2% | 75.4% | 85.2% | 85.2% |
| Percentage of spherical powder in total powder fraction (wt.%) | | 84.0% | 84.0% | 84.0% | 84.0% | 77.8% | 42.0% | 42.0% |
| Dry feel | | ⊙ | ○ | ○ | ○ | ○ | × | × |

### Examples 15 to 18

W/0 type emulsion cosmetic compositions were prepared in which one of the volatile oils listed below was added to the W/O type emulsion cosmetic composition obtained in Example 5, which had an excellent rating in each of the evaluations as shown in Table 1 and was used as a reference. The evaluations were then performed. The compounding amounts and evaluation results are shown in Table 4.

### Volatile oil

Cyclopentasiloxane: KF-995 (volatile oil, cyclic silicone oil)
Dimethicone 2: silicone KF96L-1.5cs (volatile oil, linear silicone oil)
Methyl trimethicone: TMF-1.5 (volatile oil, branched silicone oil)
Isododecane: Marukasol R (volatile oil, paraffinic hydrocarbon oil)
Hydrogenated polyisobutene: Parleam 3 (volatile oil, paraffinic hydrocarbon oil)

**[Table 4]**

| | Label name | Example 5 | Example 15 | Example 16 | Example 17 | Example 18 |
|---|---|---|---|---|---|---|
| Water | water | 34.45 | ← | ← | ← | ← |
| Humectant | glycerin | 2.00 | ← | ← | ← | ← |
| | PEG-150 | 0.50 | ← | ← | ← | ← |
| | propanediol | 7.00 | ← | ← | ← | ← |
| Chelating agent | EDTA-2Na | 0.05 | ← | ← | ← | ← |
| Preservative | phenoxyethanol | 0.50 | ← | ← | ← | ← |
| Thickening agent | magnesium aluminum silicate | 0.68 | ← | ← | ← | ← |
| | distearyldimonium chloride, isopropanol | 0.23 | ← | ← | ← | ← |
| Surfactant | PEG-10 dimethicone | 2.00 | ← | ← | ← | ← |
| | sorbitan sesquiisostearate | 1.00 | ← | ← | ← | ← |
| Oil | cyclopentasiloxane (volatile oil) | 19.90 | - | - | - | - |
| | dimethicone 2 (volatile oil) | - | 19.90 | - | - | - |
| | methyl trimethicone (volatile oil) | - | - | 19.90 | - | - |
| | isododecane (volatile oil) | - | - | - | 19.90 | - |
| | hydrogenated polyisobutene (volatile oil) | - | - | - | - | 19.90 |
| | diphenylsiloxy phenyl trimethicone | 3.00 | ← | ← | ← | ← |
| | dimethicone 1 | 1.00 | ← | ← | ← | ← |
| | triethylhexanoin | 2.00 | ← | ← | ← | ← |
| Extender pigment | talc, methicone | 3.00 | ← | ← | ← | ← |
| | mica, methicone | 2.00 | ← | ← | ← | ← |
| Color pigment | titanium oxide, aluminum hydroxide, methicone | 0.10 | ← | ← | ← | ← |
| | iron oxide, methicone | 0.10 | ← | ← | ← | ← |
| Organic pigment | Red 226, barium sulfate, talc | 0.50 | ← | ← | ← | ← |
| Feel-improving powder | methyl methacrylate crosspolymer (spherical) | 20.00 | ← | ← | ← | ← |
| Total | | 100.0 | 100.0 | 100.0 | 100.0 | 100.0 |
| Weight ratio of total powder fraction to total oil fraction | | 0.99 | 0.99 | 0.99 | 0.99 | 0.99 |
| Percentage of volatile oil in total oil fraction (wt.%) | | 76.8 | 76.8 | 76.8 | 76.8 | 76.8 |
| Ease of application | | ⊙ | ⊙ | ⊙ | ⊙ | ⊙ |
| Dry feel | | ⊙ | ⊙ | ⊙ | ⊙ | ⊙ |
| Finish | | ⊙ | ⊙ | ⊙ | ⊙ | ⊙ |
| Long wearability | | ⊙ | ⊙ | ⊙ | ⊙ | ⊙ |

A formulation example of the W/O type emulsion cosmetic composition of the present invention is described below. Note that application examples of the W/O type emulsion cosmetic composition of the present invention are not limited to the following formulation example.

### Formulation Example 1: emulsion foundation

| Classification | Ingredient name | Label name | |
|---|---|---|---|
| Water | ion-exchanged water | water | 28.15 |
| Humectant | concentrated glycerin for cosmetics | glycerin | 2.00 |
| | PEG-6000 | PEG-150 | 0.50 |
| | propanediol | propanediol | 7.00 |
| Chelating agent | Chelest 2BS | EDTA-2Na | 0.05 |
| Preservative | phenoxyethanol | phenoxyethanol | 0.50 |
| Thickening agent | Sumecton SA | magnesium aluminum silicate | 0.68 |
| | Cation DSV | distearyldimonium chloride, isopropanol | 0.23 |
| Surfactant | silicone KF-6017 | PEG-10 dimethicone | 2.00 |
| | Cosmol 182V | sorbitan sesquiisostearate | 1.00 |
| Oil | KF995 | cyclopentasiloxane (volatile oil) | 19.90 |
| | silicone KF-56A | diphenylsiloxy phenyl trimethicone | 3.00 |
| | silicone KF-96A-6cs | dimethicone | 1.00 |
| | NIKKOL Trifat S-308 | triethylhexanoin | 2.00 |
| UV absorbing agent | Parsol MCX | ethylhexyl methoxycinnamate | 2.00 |
| UV scattering agent | MT-014Z | titanium oxide, aluminum hydroxide, stearic acid | 5.00 |
| Extender pigment | SI-2 TALC JA-46R | talc, methicone | 2.00 |
| | SI-2 MICA Y-2300 | mica, methicone | 1.62 |
| Color pigment | SI-2 Ti02 CR-50 | titanium oxide, aluminum hydroxide, methicone | 5.00 |
| | SI-2 RED R-516P | iron oxide, methicone | 0.21 |
| | SI-2 YELLOW LL-100P | iron oxide, methicone | 1.11 |
| | SI-2 BLACK BL-100P | iron oxide, methicone | 0.06 |
| Feel-improving powder | GMX-0810 | methyl methacrylate crosspolymer (spherical powder) | 15.00 |
| Total | | | 100.00 |

The W/O type emulsion cosmetic composition of the present invention is not limited to the Examples and Formulation Example described above, and various modifications, changes, and improvements may be made to any of the disclosed elements (including elements described in the Claims or Description) within the scope of the present invention and based on the technical spirit of the present invention. Furthermore, various combinations, replacements, and selections of any of the disclosed elements are possible within the scope of the Claims of the present invention.

With regard to the numerical ranges described herein, it is to be understood that any number or range included in the range is specifically disclosed herein even if not explicitly stated.

## Claims

1. A W/O type emulsion cosmetic composition comprising a volatile oil and a spherical powder, **characterized in that**
a weight ratio between a total oil fraction and a total powder fraction (total powder fraction/total oil fraction) is 0.75 or greater and 1.40 or less, and
after being applied to a skin, the W/O type emulsion cosmetic composition becomes a powder as a result of volatilization of a liquid.

2. The W/O type emulsion cosmetic composition according to Claim 1, **characterized in that** the volatile oil includes at least one of cyclic silicone oils, linear or branched silicone oils, and paraffinic hydrocarbon oils.

3. The W/O type emulsion cosmetic composition according to Claim 1 or 2, **characterized in that** the spherical powder has a volume average particle diameter of 5 µm or greater and 20 µm or less.

4. The W/O type emulsion cosmetic composition according to any one of Claims 1 to 3, **characterized in that** a percentage of the volatile oil in the total oil fraction (volatile oil/total oil fraction) is 30 wt.% or greater.

5. The W/0 type emulsion cosmetic composition according to any one of Claims 1 to 4, **characterized in that** a percentage of the spherical powder in the total powder fraction (spherical powder/total powder fraction) is 45 wt.% or greater.
